# FASCICULE DE BREVET EUROPEEN

(11) **EP 2 023 840 B1**
(45) Date de publication et mention de la délivrance du brevet: **26.01.2011**
(21) Numéro de dépôt: 07729349.6
(22) Date de dépôt: 22.05.2007
(51) Int. Cl.: A61B 17/88, A61C 8/00, B25B 23/14

(54) **OUTIL DYNAMOMETRIQUE A USAGE MEDICAL**
DYNAMOMETRISCHES WERKZEUG ZUR MEDIZINISCHEN VERWENDUNG
DYNAMOMETRIC TOOL FOR MEDICAL USE

(30) Priorité: 26.05.2006 CH 857062006
(43) Date de publication de la demande: 18.02.2009
(73) Titulaire: Hader SA, 2300 La Chaux-de-Fonds (CH)
(72) Inventeur: GROSS, Silver, 2300 Chaux-de-Fonds (CH)
(74) Mandataire: GLN
(86) Numéro de dépôt international: PCT/EP2007/054906
(87) Numéro de publication internationale: WO 2007/137961

(56) Documents cités:
- DE-C1- 10 043 787
- US-A- 500 721
- US-A- 5 368 480

## Description

### Domaine technique

La présente invention se rapporte au domaine des outils à usage médical. Elle concerne, plus particulièrement, un outil dynamométrique destiné à serrer et à desserrer des vis ou divers objets comportant un pas de vis, au cours d'une intervention chirurgicale.

Il est, en effet, particulièrement important d'éviter d'appliquer des couples de serrage non contrôlés, par exemple dans le cas où une plaque est fixée sur un os pour réparer une fracture. Si le serrage appliqué est excessif, cela peut conduire à écraser l'os et à l'endommager davantage.

### Etat de la technique

Le document US 5,368, 480 décrit un outil dynamométrique.

Son contenu forme la base de préambule de le présente revendication 1.

On connaît, dans l'état de la technique, des outils dynamométriques pour le vissage, et parfois le dévissage, de différents objets, notamment de vis pour la fixation d'éléments de reconstruction dans la chirurgie réparatrice. A titre d'exemple d'application, des plaques peuvent être vissées dans des os fracturés en vue de faciliter leur réparation. Un tel outil comprend:
- un corps de préhension pour que le chirurgien puisse le manipuler, et
- un porte-instrument pour être rendu solidaire en rotation d'un instrument conformé pour coopérer avec l'objet à visser.

Dans certains outils de ce type, le porte-instrument se prolonge par un arbre pivotant dans le corps de préhension. Il est relié à friction au corps de préhension au moyen d'un organe ressort disposé entre l'arbre et le corps de préhension. La friction est assurée par deux dentures Breguet, c'est-à-dire des dentures de chant en forme de scie, l'une étant solidaire du porte-instrument et l'autre étant solidaire en rotation du corps de préhension. Des ressorts pressent les deux dentures Breguet l'une contre l'autre de manière à rendre solidaires en rotation le porte-instrument et le corps de préhension. Quand le couple de serrage est supérieur aux frictions imposées entre les deux dentures Breguet par les ressorts, celles-ci frottent l'une contre l'autre et s'échappent. Le porte-instrument n'est alors plus entraîné en rotation par le corps de préhension. Le couple de serrage maximal applicable peut être réglé en modulant la pression exercée par les ressorts.

Les frictions créées entre les dentures sont particulièrement importantes et il est nécessaire, pour obtenir une précision et une longévité acceptables de l'outil, que les dentures Breguet soient métalliques. Elles, ainsi que les ressorts, sont réalisées en un acier inoxydable, permettant une utilisation chirurgicale aussi hygiénique que possible. Il est toutefois nécessaire de graisser les parties métalliques en friction, ce qui n'est pas très satisfaisant d'un point de vue sanitaire, puisqu'un risque de coulée de graisse à l'extérieur de l'outil existe lors des opérations de stérilisation. En outre, la précision d'un tel outil est peu satisfaisante (+/- 10%) et il est nécessaire d'effectuer régulièrement des calibrations.

De plus, lorsque le couple de serrage maximal est atteint et que les dentures s'échappent l'une de l'autre, cela provoque des sauts dans la direction longitudinale de l'outil, ce qui n'est pas agréable pour le chirurgien et qui peut lui faire faire un geste maladroit.

Par ailleurs, les matériaux utilisés pour la réalisation de cet outil le rendent lourd et peu pratique.

La présente invention a donc pour but de proposer un outil dynamométrique exempt des inconvénients susmentionnés et qui, en particulier, est précis, léger, facile à manipuler. Particulièrement, lorsque le couple de serrage maximal est atteint, aucune secousse parasite n'est ressentie par l'utilisateur. Divulgation de l'invention

De façon plus précise, l'invention concerne un outil dynamométrique à usage médical comportant:
- un corps de préhension creux,
- un porte-instrument pour être rendu solidaire en rotation d'un instrument conformé pour coopérer avec un objet à visser, ledit porte-instrument se prolongeant par un arbre pivotant à l'intérieur du corps de préhension.

Le porte-instrument est relié à friction au corps de préhension au moyen d'un organe ressort disposé entre l'arbre et le corps de préhension.

Selon l'invention, l'organe ressort comporte une pluralité de lamelles déformables élastiquement selon une direction essentiellement radiale.

Selon un mode de réalisation avantageux, l'organe ressort comporte une pluralité de lamelles disposées principalement selon des directions non radiales.

### Brève description des dessins

D'autres détails apparaîtront plus clairement à la lecture de la description qui suit, faite en regard des dessins annexés, dans lesquels :
- la figure 1 est une vue en coupe longitudinale du dispositif selon l'invention,
- la figure 2 est une vue en coupe transversale du corps de préhension, comportant un plan rapproché d'une zone de cette partie,
- la figure 3 illustre particulièrement un mode de réalisation d'un organe ressort particulièrement adapté pour la mise en oeuvre de l'outil selon l'invention, et
- les figures 4a et 4b présentent, respectivement en coupe et vue de dessus, un deuxième mode de réalisation d'un organe ressort.

### Mode(s) de réalisation de l'invention

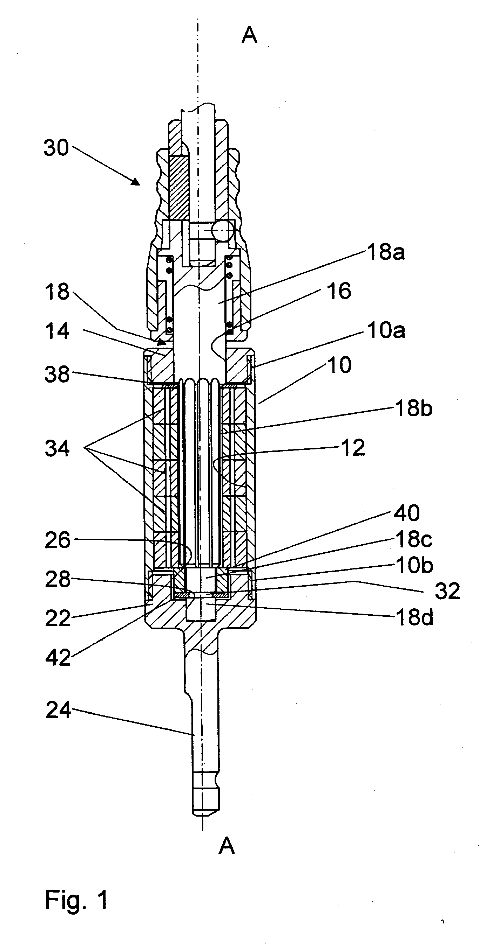

La figure 1 montre un corps de préhension 10, d'axe longitudinal AA. Selon cet axe, le corps 10 est traversé par un canal cylindrique 12. Une première extrémité du corps de préhension est dotée d'un pas de vis 10a pour coopérer avec un premier bouchon 14. Ce dernier comporte un trou 16 destiné à laisser passer un arbre 18 qui sera décrit plus en détail ci-après.

La deuxième extrémité du corps de préhension est également dotée d'un pas de vis 10b pour coopérer avec un deuxième bouchon 22. Dans le mode de réalisation illustré, celui-ci se prolonge par une tige 24 pouvant être reliée à un appareil non représenté, muni d'un moteur pour mettre la tige en rotation. En variante, un manche peut être fixé autour de la tige 24 pour prolonger le corps de préhension 10 et faciliter la manipulation de l'outil.

Du côté de l'intérieur du corps 10, les parois du deuxième bouchon 22 forment un logement 26 dont le fond présente un orifice cylindrique 28 destiné à recevoir l'extrémité de l'arbre 18.

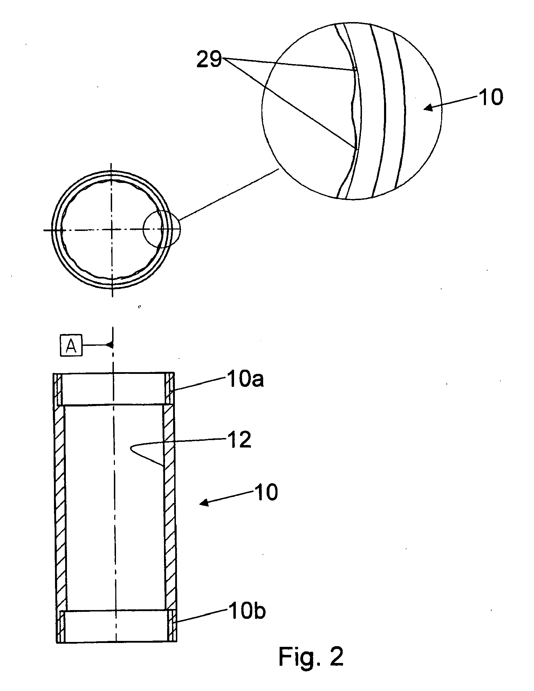

La figure 2 illustre en détail un exemple de réalisation de la structure du canal 12 dont la paroi intérieure présente une succession de creux 29, typiquement de forme générale cylindrique, orientés selon l'axe AA. Les creux 19 occupent toute la longueur du canal comprise entre les deux pas de vis 10a et 10b.

On a également représenté sur la figure 1 un porte-instrument 30 de type connu de l'homme du métier, susceptible d'être rendu solidaire en rotation d'un instrument conformé pour coopérer avec un objet à visser. La partie du porte-instrument assurant la liaison avec l'instrument ne fait pas en soi partie de l'invention et ne sera pas décrite en détail.

Le porte-instrument 30 se prolonge par l'arbre 18 mentionné précédemment. Celui-ci est dimensionné de manière à pouvoir traverser le trou 16, prendre place dans le canal 12 du corps de préhension 10, tandis que son extrémité libre prend place dans le logement 26 et dans l'orifice 28. Plus précisément, l'arbre 18 comporte une première portion 18a ajustée à la dimension du trou 16. Puis, en s'éloignant de la partie du porte-instrument destinée à recevoir un instrument, une deuxième portion 18b destinée à être logée dans le canal 12. La deuxième portion 18b est structurée de manière à présenter une section typiquement en étoile, et forme ainsi un organe mâle pouvant être lié en rotation avec un organe femelle de forme correspondante. Enfin, l'arbre se termine par une troisième portion 18c destinée à prendre place dans le logement 26 et par une quatrième portion 18d ajustée aux dimensions de l'orifice 28. Ces deux dernières portions sont séparées par une gorge annulaire 32 orthogonale à l'axe longitudinal de l'arbre.

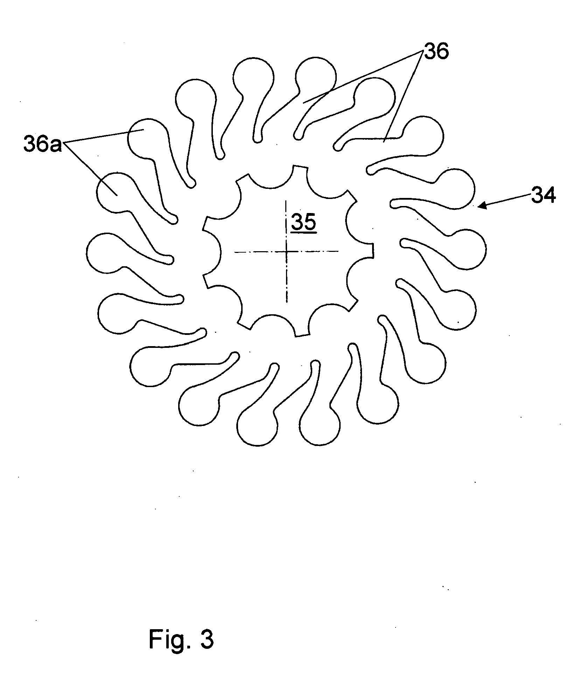

Le porte-instrument 30 est destiné à être monté pivotant, à friction, dans le canal 12. Selon un aspect important de l'invention, la friction est assurée par au moins un organe ressort 34 dont un exemple est illustré sur la figure 3.

Cet organe ressort 34 est de forme générale cylindrique et comporte, en son centre, une ouverture 35 structurée de manière à présenter une section typiquement en étoile, et forme ainsi un organe femelle conformé pour être monté sans jeu et solidaire en rotation sur la portion 18b de l'arbre 18. La forme en étoile proposée n'est qu'un exemple, d'autres types de fentes, de structures ou d'ergots peuvent être utilisés, l'essentiel étant que ces éléments soient solidaires en rotation.

L'organe ressort 34 comporte une pluralité de lamelles 36 déformables élastiquement selon une direction essentiellement radiale. Ces lamelles 36 sont orientées vers l'extérieur, principalement selon des directions non radiales et se terminent, dans un mode de réalisation préféré, par une portion cylindrique 36a sensiblement orthogonale au plan général de l'organe ressort.

L'organe ressort 34 est destiné à être monté sur l'arbre 18, interposé entre ce dernier et la paroi du canal 12. Les portions cylindriques 36a sont donc définies de manière à coopérer avec les creux 29 du canal. D'autres formes peuvent être choisies tant que les extrémités des lamelles 36 sont capables de coopérer avec les structures de la paroi du canal 12 pour créer une friction.

En terme de dimensions, l'homme du métier pourra choisir la longueur, l'orientation, le nombre et l'épaisseur (selon une direction perpendiculaire au plan général de l'organe ressort) des lamelles 36 de l'organe ressort 34 de manière à définir la friction exercée entre l'organe ressort 34 et le corps de préhension 10. Ces différents paramètres permettent de déterminer la valeur du couple maximal de serrage applicable.

Le nombre de ressorts 34 disposés sur l'arbre 18 peut également être modulé pour ajuster le couple maximal de serrage. Toutefois, on comprendra ci-après, en décrivant le montage de l'outil, que presque toute la longueur de la deuxième portion 18b de l'arbre doit être occupée par les organes ressort. Ainsi, pour faire varier le nombre d'organes ressort disposés sur l'arbre, il est possible de prévoir des portes-instruments et des corps de préhension de longueur adaptée.

Une autre solution avantageuse consiste à remplacer un organe ressort par un dispositif de calage, présentant d'une structure similaire, mais ne comportant pas de point de contact avec le corps de préhension. Ce dispositif peut consister en un organe ressort duquel les portions cylindriques auront été supprimées. Une simple douille peut aussi remplacer un organe ressort pour diminuer le couple maximal de serrage. De préférence, les organes ressorts sont centrés sur la portion 18b, c'est-à-dire que les dispositifs de calage sont également répartis de part et d'autre des organes ressorts. L'homme du métier pourra également prévoir des organes ressorts 34 d'épaisseurs différentes pour donner d'autres possibilités d'ajustement. Les éventuelles opérations de réglage du couple maximal de serrage sont effectuées en usine. De préférence, l'utilisateur ne peut, pour des raisons de sécurité et de traçabilité des opérations, moduler lui-même la valeur du couple maximal de serrage.

L'organe ressort est avantageusement réalisé en une matière plastique autolubrifiante et résistant aux traitements habituels de stérilisation, thermiques et par rayonnement. Différents tests ont permis de démontrer que les polymères du type polyéther-éther-cétone (connu sous le nom de PEEK) présentaient les caractéristiques requises. On pourra choisir, plus précisément du PEEK 151 G.

Typiquement, des organes ressort tels que décrits ci-dessus et réalisés en PEEK permettent d'obtenir des valeurs de couple de serrage de l'ordre de quelques N.m, typiquement comprises entre 1 et 10 N.m.

Ainsi, pour assembler l'outil tel qu'illustré sur la figure 1, les opérations suivantes sont effectuées, de préférence en usine par un opérateur. Le premier bouchon 14 est d'abord fixé fermement sur le corps de préhension et l'arbre du porte-instrument est introduit dans le trou 16. Ensuite, une bague 38 est montée sur l'arbre 18 de manière à définir une surface d'appui précise pour les organes ressorts 34 dont le nombre choisi est interposé entre l'arbre 18 et le canal 12. Les organes ressorts sont donc solidaires en rotation de l'arbre 18 et en contact avec la paroi du canal 12.

Puis, une cale 40 est disposée sur l'arbre 18, au niveau de la troisième portion 18c. Cette cale 40 doit être appuyée sur le dernier organe ressort disposé dans le canal 12 et non pas sur un éventuel seuil situé entre les deuxième et troisième portions. La cale 40 est dimensionnée pour occuper exactement l'espace compris entre le dernier ressort et la gorge 32. Un organe de blocage 42 est alors disposé dans cette gorge 32 pour positionner axialement les éléments déjà mis en place. Enfin, le deuxième bouchon 22 et la tige 24 sont vissés à l'extrémité du corps de préhension 10 pour assurer le maintien de l'ensemble ainsi formé.

La bague 38 d'une part, la cale 40 et l'organe de blocage 42 d'autre part, permettent de positionner longitudinalement le corps de préhension par rapport au porte-instrument et de limiter fortement les frottements entre les bouchons et les organes ressorts afin de pouvoir parfaitement contrôler le réglage du couple maximal de serrage.

On notera que le couple de serrage des bouchons doit être supérieur au couple de serrage maximal déterminé. De préférence, les bouchons peuvent être soudés au corps de préhension 10.

Ainsi est obtenu un outil dont le réglage du couple maximal de serrage peut être particulièrement précis, avec une durée de vie améliorée. Des essais ont permis de montrer que la précision atteinte était de l'ordre de 3% pour 10 000 déclenchements. On définit un déclenchement comme étant le moment où le corps de préhension se désolidarise du porte-instrument, passant d'une première à une deuxième position relative de l'un de ces éléments par rapport à l'autre. De plus, grâce à la déformation radiale des organes ressorts, aucun mouvement parasite n'est à déplorer lorsque le couple de serrage maximal est atteint. L'outil n'a pas besoin d'être lubrifié et peut être étanche, évitant tout risque de contamination du patient:

En outre, grâce à l'orientation des lamelles, la friction maximale qui s'exerce entre les organes ressort et la paroi du canal est différente dans le sens du vissage et dans celui du dévissage. L'orientation des lamelles est choisie de manière à ce qu'un même outil puisse être utilisé, sans modifier le réglage du couple maximal de serrage, pour dévisser immédiatement l'objet qui vient d'être vissé.

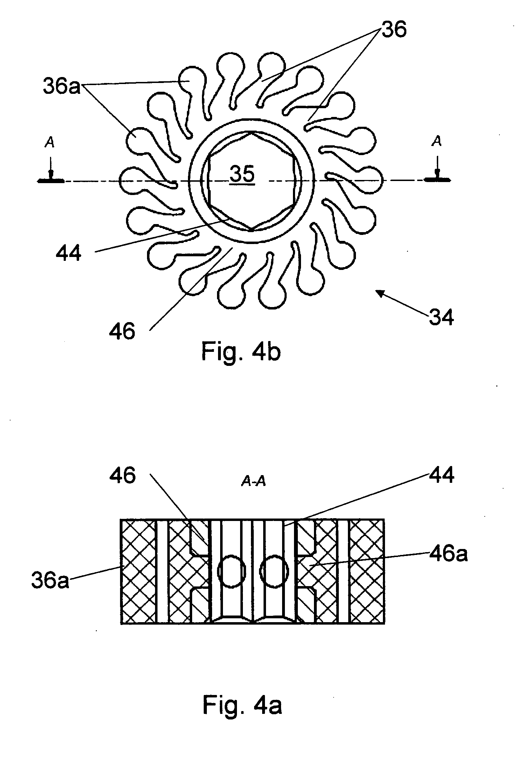

Pour des couples importants, un organe ressort selon un deuxième mode de réalisation est proposé. Pour améliorer la liaison avec l'arbre, l'organe ressort tel qu'illustré sur la figure 4 comporte un moyeu 44 réalisé en un matériau non déformable élastiquement, par exemple de type métallique, tel qu'un acier inox. Comme ci-dessus, le moyeu présente une ouverture 35 structurée de manière à former un organe femelle conformé pour être monté sans jeu et solidaire en rotation sur la portion 18b de l'arbre 18.

L'organe ressort comporte en outre une bague 46 portant les lamelles 36. Cette bague est réalisée dans un des matériaux proposés ci-dessus pour l'organe ressort. La bague est agencée autour du moyeu, de manière solidaire.

La bague peut être surmoulée sur le moyeu. Pour ce faire, le moyeu présente avantageusement des canaux le traversant transversalement et débouchant dans l'ouverture 35. Ces canaux sont utilisés pour injecter la matière plastique permettant de réaliser la bague. Il résulte de ce procédé de fabrication que la bague présente des tiges 46a logées sans jeu dans les canaux et qui assurent une excellente liaison, particulièrement rigide, entre le moyeu et la bague. En outre, l'étape d'injection ainsi réalisée ne laisse aucune trace sur les parties supérieure et inférieure des organes ressorts, ce qui évite toute opération de retouche.

La description ci-dessus n'a été donnée qu'à titre d'illustration de l'invention et n'en constitue pas une limitation. Ainsi, sans sortir du cadre de l'invention, l'homme du métier pourra également choisir de disposer des organes ressorts toujours sur l'arbre du porte-instrument, mais de manière à ce que les organes ressorts soient solidaires en rotation du corps de préhension et frottent sur des structures que comporte l'arbre. Ainsi, dans une telle alternative, le canal peut typiquement présenter une section polygonale avec laquelle coopèrent les organes ressorts. Ces derniers présentent des lamelles qui sont orientées vers l'intérieur et qui coopèrent avec des structures de forme adaptée ménagées dans l'arbre. Un organe ressort en deux parties pourrait très bien être adapté à ce mode de réalisation.

## Revendications

1. Outil dynamométrique à usage médical comportant:
- un corps de préhension (10) creux,
- un porte-instrument (30) pour être rendu solidaire en rotation d'un instrument conformé pour coopérer avec un objet à visser, ledit porte-instrument se prolongeant par un arbre (18) pivotant à l'intérieur du corps de préhension,
ledit porte-instrument (30) étant relié à friction audit corps de préhension au moyen d'un organe ressort (34) disposé entre ledit arbre (18) et ledit corps de préhension (10),
**caractérisé en ce que** ledit organe ressort (34) comporte une pluralité de lamelles (36) déformables élastiquement selon une direction essentiellement radiale
de sorte que le porte-instument n'est alors plus entrainé en rotation par le corps de préhension quand le couple de
serrage maximal est atteint.

2. Outil selon la revendication 1, **caractérisé en ce que** ledit organe ressort (34) comporte une pluralité de lamelles (36) disposées principalement selon des directions non radiales.

3. Outil selon l'une des revendications 1 et 2, **caractérisé en ce que** ledit organe ressort comporte un moyeu réalisé en un matériau non déformable élastiquement et une bague portant les lamelles et montée solidairement autour du moyeu.

4. Outil selon la revendication 3, **caractérisé en ce que** ledit moyeu est doté de canaux le traversant transversalement et **en ce que** ladite bague présente des tiges logées sans jeu dans les canaux.

5. Outil selon l'une des revendications précédentes, **caractérisé en ce que** l'intérieur dudit corps de préhension est de forme cylindrique, **en ce que** ledit organe ressort est solidaire en rotation dudit porte-instrument et **en ce que** lesdites lamelles (36) sont orientées vers l'extérieur.

6. Outil selon l'une des revendications précédentes, **caractérisé en ce que** la surface intérieure dudit corps de préhension (10) présente une structure conformée pour coopérer avec les extrémités des lamelles (36) de l'organe ressort (34).

7. Outil selon la revendication 6, **caractérisé en ce que** les lamelles de l'organe ressort se terminent par une portion cylindrique (36a) destinées à être sensiblement parallèle audit arbre et **en ce que** la surface intérieure dudit corps de préhension (10) présente une succession de creux de forme générale cylindrique, destinés à être sensiblement parallèles audit arbre et conformés pour coopérer avec lesdites portions cylindriques (36a).

8. Outil selon l'une des revendications 1 et 2, **caractérisé en ce que** ledit organe ressort (34) est solidaire en rotation dudit corps de préhension (10) et **en ce que** lesdites lamelles (36) sont orientées vers l'intérieur.

9. Outil selon la revendication 8, **caractérisé en ce que** la surface dudit arbre (18) présente une structure conformée pour coopérer avec les extrémités desdites lamelles (36).

10. Outil selon la revendication 9, **caractérisé en ce que** les lamelles (36) de l'organe ressort (34) se terminent par une portion cylindrique (36a) destinée à être sensiblement parallèle audit arbre et **en ce que** la surface dudit arbre présente une succession de creux de forme générale cylindrique, destinés à être sensiblement parallèles audit arbre et conformés pour coopérer avec les portions cylindriques de l'organe ressort.

11. Outil selon l'une des revendications précédentes, **caractérisé en ce que** ledit organe ressort est réalisé en PEEK.

## Claims

1. A power torque tool for medical use including:
- a hollow gripping body (10),
- an instrument-holder (30) so as to be interdependent in rotation with an instrument conformed for co-operating with an object to be screwed, said instrument-holder extending with a shaft (18) pivoting inside the gripping body,
said instrument-holder (30) being frictionally connected by means of a spring member (34) arranged between said shaft (18) and said gripping body (10),
**characterized in that** said spring member (34) includes a plurality of elastically deformable strips (36) along an essentially radial direction so that the instrument-holder is then no longer driven into rotation by the gripping body when the maximum tightening torque is reached.

2. The tool according to claim 1, **characterized in that** said spring member (34) includes a plurality of strips (36) mainly arranged along non-radial directions.

3. The tool according to any of claims 1 and 2, **characterized in that** said spring member includes a hub made in an elastically non-deformable material and a ring bearing strips and interdependently mounted around the hub.

4. The tool according to claim 3, **characterized in that** said hub is provided with channels crossing it transversely and **in that** said ring has rods accommodated in the channels without any play.

5. The tool according to any of the preceding claims, **characterized in that** the interior of said gripping body is of cylindrical shape, **in that** said spring member is interdependent in rotation with said instrument holder and **in that** said strips (36) are oriented outwards.

6. The tool according to any of the preceding claims, **characterized in that** the interior surface of said gripping body (10) has a structure conformed so as to co-operate with the ends of the strips (36) of the spring member (34).

7. The tool according to claim 6, **characterized in that** the strips of the spring member end with a cylindrical portion (36a) intended to be substantially parallel to said shaft and **in that** the interior surface of said gripping body (10) has a succession of recesses of a general cylindrical shape, intended to be substantially parallel to said shaft and conformed so as to co-operate with said cylindrical portions (36a).

8. The tool according to any of claims 1 and 2, **characterized in that** said spring member (34) is interdependent in rotation with said gripping body (10) and **in that** said strips (36) are oriented inwards.

9. The tool according to claim 8, **characterized in that** the surface of said shaft (18) has a structure conformed for co-operating with the ends of said strips (36).

10. The tool according to claim 9, **characterized in that** the strips (36) of the spring member (34) end with a cylindrical portion (36a) intended to be substantially parallel to said shaft and **in that** the surface of said shaft has a succession of recesses of a general cylindrical shape, intended to be substantially parallel to said shaft and conformed so as to co-operate with the cylindrical portions of the spring member.

11. The tool according to any of the preceding claims, **characterized in that** said spring member is made in PEEK.

## Patentansprüche

1. Dynamometrisches Werkzeug zur medizinischen Anwendung, das umfasst:
- einen hohlen Greifkörper (10),
- einen Instrumentenhalter (30) zur rotierenden Verbindung mit einem Instrument, das ausgebildet ist, um mit einem zu schraubenden Gegenstand zusammenzuarbeiten, wobei sich der Instrumentenhalter durch eine im Greifkörper drehende Welle (18) verlängert,
wobei der Instrumentenhalter (30) reibend mit dem Greifkörper mittels eines Federorgans (34) verbunden ist, das zwischen der Welle (18) und dem Greifkörper (10) angeordnet ist,
**dadurch gekennzeichnet, dass** das Federorgan (34) eine Mehrzahl von Lamellen (36) aufweist, die in einer im Wesentlichen radialen Richtung elastisch deformierbar sind, so dass der Instrumentenhalter dann nicht mehr rotierend vom Greifkörper angetrieben wird, wenn das maximale Anziehdrehmoment erreicht ist.

2. Werkzeug nach Anspruch 1, **dadurch gekennzeichnet, dass** das Federorgan (34) eine Mehrzahl von Lamellen (36) aufweist, die im Wesentlichen gemäß nicht radialer Richtungen angeordnet sind.

3. Werkzeug nach einem der Ansprüche 1 und 2, **dadurch gekennzeichnet, dass** das Federorgan eine Nabe aufweist, die aus einem nicht elastisch deformierbaren Material gefertigt ist und einen Ring, der die Lamellen trägt und um die Nabe verbunden montiert ist.

4. Werkzeug nach Anspruch 3, **dadurch gekennzeichnet, dass** die Nabe mit Kanälen ausgestattet ist, die sie quer durchqueren und dass der Ring Stäbe aufweist, die ohne Spiel in den Kanälen untergebracht sind.

5. Werkzeug nach einem der vorangehenden Ansprüche, **dadurch gekennzeichnet, dass** das Innere des Greifkörpers zylindrischer Form ist, dass das Federorgan rotierend mit dem Instrumentenhalter verbunden ist und dass die Lamellen (36) nach außen ausgerichtet sind.

6. Werkzeug nach einem der vorangehenden Ansprüche, **dadurch gekennzeichnet, das** die innere Oberfläche des Greifkörpers (10) eine Struktur aufweist, die ausgebildet ist, um mit den Enden der Lamellen (36) des Federorgans (34) zusammenzuarbeiten.

7. Werkzeug nach Anspruch 6, **dadurch gekennzeichnet, dass** die Lamellen des Federorgans durch einen zylindrischen Abschnitt (36a) beendet werden, der dazu bestimmt ist, etwa parallel zur Welle zu sein und dass die innere Oberfläche des Greifkörpers (10) eine Abfolge von Hohlräumen allgemein zylindrischer Form aufweist, die dazu bestimmt sind, etwa parallel zur Welle zu sein und ausgebildet sind, um mit den zylindrischen Abschnitten (36a) zusammenzuarbeiten.

8. Werkzeug nach einem der Ansprüche 1 und 2, **dadurch gekennzeichnet, dass** das Federorgan (34) rotierend mit dem Greifkörper (10) verbunden ist und dass die Lamellen (36) nach innen ausgerichtet sind.

9. Werkzeug nach Anspruch 8, **dadurch gekennzeichnet, dass** die Oberfläche der Welle (18) eine Struktur aufweist, die ausgebildet ist, um mit den Enden der Lamellen (36) zusammenzuarbeiten.

10. Werkzeug nach Anspruch 9, **dadurch gekennzeichnet, dass** die Lamellen (36) des Federorgans (34) durch einen zylindrischen Abschnitt (36a) beendet werden, der dazu bestimmt ist, etwa parallel zur Welle zu sein und dass die Oberfläche der Welle eine Abfolge von Hohlräumen allgemein zylindrischer Form aufweist, die dazu bestimmt sind, etwa parallel zur Welle zu sein und ausgebildet sind, um mit den zylindrischen Abschnitten des Federorgans zusammenzuarbeiten.

11. Werkzeug nach einem der vorangehenden Ansprüche, **dadurch gekennzeichnet, dass** das Federorgan aus PEEK hergestellt ist.
